(19) Europäisches Patentamt
European Patent Office
Office européen des brevets

(11) EP 1 074 258 A2

(12) EUROPEAN PATENT APPLICATION

(43) Date of publication:
**07.02.2001 Bulletin 2001/06**

(51) Int. Cl.7: **A61K 31/519**, A61P 27/02, A61P 27/06

(21) Application number: **00306235.3**

(22) Date of filing: **21.07.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **28.07.1999 US 146095**

(71) Applicant: **Pfizer Products Inc.**
**Groton, Connecticut 06340 (US)**

(72) Inventor: **Laties, Alan Malev**
**Philadelphia, Pennsylvania 19119 (US)**

(74) Representative:
**McMunn, Watson Palmer et al**
**Pfizer Limited**
**Patents Department**
**Ramsgate Road**
**Sandwich, Kent CT13 9NJ (GB)**

# (54) Methods and compositions for treating diseases and conditions of the eye

(57) Methods for the prevention and treatment of diseases and conditions of the eye including, but are not limited to: central retinal artery occlusion; central retinal vein occlusion; optic neuropathy including, but not limited to, anterior ischemic optic neuropathy and glaucomatous optic neuropathy; and macular (dry) degeneration are disclosed. These methods comprise administering to a patient a prophylactically or therapeutically effective amount of a cyclic guanosine 3',5'-monophosphate phosphodiesterase type 5 inhibitor. Pharmaceutical compositions and dosage forms comprising cyclic guanosine 3',5'-monophosphate phosphodiesterase type 5 inhibitors are also disclosed.

EP 1 074 258 A2

## Description

Field of Invention

This invention relates to methods of using, and compositions comprising, cyclic guanosine 3',5'-monophosphate phosphodiesterase type 5 inhibitors. The methods and compositions of the invention are useful in the prevention and treatment of diseases and conditions of the eye including, but not limited to, central retinal artery occlusion, central retinal vein occlusion, optic neuropathy including anterior ischemic optic neuropathy and glaucomatous optic neuropathy, and macular (dry) degeneration.

Background of the Invention

The flow of blood delivers oxygen, sugar, amino acids, and other essential compounds to the eye. Temporary or permanent damage to the eye can thus result when this "vascular nutrition" is insufficient or is hindered by the obstruction or constriction of blood vessels.

An optic neuropathy (*i.e*., a disease or damage of the optic nerve) underlies the loss of vision characteristic of several forms of glaucoma. It is widely accepted that glaucomatous optic neuropathy is usually caused by unacceptably high intraocular pressure (IOP). The upper limit of normal human IOP, which depends on the inflow and outflow of aqueous humor, is variously defined to be about 20 to about 22 mm Hg above atmospheric pressure. Cioffi, G.A. and Van Buskirk, E.M., *Textbook of Ophthalmology*, Wright, K.W. ed., p. 563 (Williams & Wilkins, 1997). Current methods of treating glaucoma are thus directed at decreasing IOP. *The Merck Manual* 735-736 ($17^{th}$ ed. 1999). Drugs currently used for the treatment of glaucoma either increase outflow or decrease inflow of the aqueous humour. See, *e.g*., *Physicians' Desk Reference for Ophthalmology* 10-12 ($27^{th}$ ed. 1999).

Although high IOP reportedly causes most cases of glaucomatous optic neuropathy, an inadequate supply of blood to the optic nerve reportedly causes glaucomatous optic neuropathy in some cases. *The Merck Manual* 734 ($17^{th}$ ed. 1999); *see also,* Delaey, C. and Van de Voorde, J., *Invest*. *Ophthal*. *& Vis*. *Sci*. 39(9):1642-1646 (1998). Despite such reports, the approximately one-third of patients with open-angle glaucoma who have pressures in a normal range are treated with agents that lower IOP. Unfortunately, in cases wherein glaucomatous optic neuropathy results from insufficient vascular nourishment (*i.e*., vascular malnourishment) of the optic nerve, reduction of IOP can have inadequate therapeutic effect.

It has recently been reported that the systemic administration of a nitric oxide (NO) donor can slow the progression of glaucomatous optic neuropathy. *See, e.g*., Afshari, N.A., *et al*., *Invest. Ophthalmol*. *Vis. Sci*. 38(Suppl.):S277 (1997). At least two explanations have been proposed for this observation, both of which are based on the ability of nitric oxide (NO) to activate the enzyme guanylate cyclase and thereby increase levels of cyclic guanosine monophosphate (cGMP), a compound which induces the relaxation of smooth muscle cells.

Relating to the front of the eye, a first theory is that the cells that make up the trabecular meshwork relax when exposed to NO in much the same way as do smooth muscle cells. Thus it has been proposed that NO can reduce IOP by relaxing the trabecular meshwork, which allows drainage of aqueous humour when distended. Grierson, I., *The Lancet* 347:1781-1782 (1996). This theory is supported by the well documented ability of NO donors to lower IOP in animals and man. Delaey, C. and Van de Voorde, J., *Invest. Ophthal*. *& Vis*. *Sci*. 39(9):1642-1646 (1998).

It has also been postulated that NO can slow the progression of glaucomatous optic neuropathy by inducing dilation of blood vessels in the eye. This theory is supported by a recent report that the administration of the NO donor 5-isosorbide mononitrate increases blood flow to the optic nerve head in humans. Grunwald, J.E., *et al*, *British J*. *Ophthal*. 83(2):162-167 (1999). The authors of that research proposed that the vasodilatation caused by NO can improve perfusion of the optic nerve. At the same time, however, they noted that NO is known to have a neurotoxic potential, and that there is concern that increased NO synthase immunoreactivity at the optic nerve head may actually correlate with disease progression in glaucoma. *Id*.; *see also*, Goldstein, I.M., *et al*., *Vision Res*. 36(18):2979-2994 (1996), and Neufeld, A.H., *et al*., *Arch Ophthaimol*. 115:397-503 (1997).

In sum, research has suggested that while administration of NO can slow the progress of glaucomatous optic neuropathy, it may not always be safe. A safe and effective method of treating or preventing diseases and conditions of the eye such as glaucomatous optic neuropathy is thus desired. Such a method preferably allows treatment of ischemic conditions, wherein damage, blockage, or constriction of a blood vessel to the eye has or will occur.

U.S. Patent No. 5,250,534 discloses a class of cyclic guanosine 3',5'-monophosphate (cGMP) phosphodiesterase type 5 (PDE5) inhibitors potentially useful in the treatment of, for example, conditions of reduced blood vessel patency and glaucoma. One member of this class is sildenafil, which has two chemical names: 1-[[3-(6,7-dihydro-1 methyl-7-oxo-3-propyl-1*H-pyrazolo*[4,3-*d*]pynmidin-5-yl)-4-ethoxyphenyl]sulfonyl]-4-methylpiperazine and 5-[2-ethoxy-5-(4-methylpiperazinylsulphonyl)phenyl]-1-methyl-3-n-propyl-1,6-dihydro-7H-pyrazolo[4,3-d]pyrimidin-7-one. Sildenafil citrate is sold under the tradename Viagra® by Pfizer Inc. and is indicated for the treatment of erectile dysfunction. *Physicians' Desk Reference* 2424-2426 ($53^{rd}$

ed. 1999). By slowing the rate of cGMP breakdown, sildenafil enhances the vasodilatory effect of naturally produced NO.

Summary of the Invention

This invention is directed to novel methods and compositions for treating and preventing acute, sub-acute, and chronic diseases and conditions of the eye. Examples of acute, sub-acute, and chronic diseases and conditions of the eye include, but are not limited to: central retinal or posterior ciliary artery occlusion: central retinal vein occlusion; optic neuropathy including, but not limited to, anterior ischemic optic neuropathy and glaucomatous optic neuropathy; and macular (dry) degeneration.

A first embodiment of the invention encompasses a method of treating or preventing central retinal or posterior ciliary artery occlusion which comprises administering to a patient in need of such treatment or prevention a therapeutically effective amount of a cyclic guanosine 3',5'-monophosphate phosphodiesterase type 5 inhibitor.

A second embodiment of the invention encompasses a method of treating or preventing central retinal vein occlusion which comprises administering to a patient in need of such treatment or prevention a therapeutically effective amount of a cyclic guanosine 3',5'-monophosphate phosphodiesterase type 5 inhibitor.

A third embodiment of the invention encompasses a method of treating or preventing optic neuropathy which comprises administering to a patient in need of such treatment or prevention a therapeutically effective amount of a cyclic guanosine 3',5'-monophosphate phosphodiesterase type 5 inhibitor.

Patients in need of treatment or prevention of optic neuropathy include, but are not limited to: patients with elevated intraocular pressure; patients greater than about 50 years of age; patients with family histories of optic neuropathy; patients with hypertension; patients with diabetes; patients with family histories of diabetes or heart disease; patients who have used, or are currently using, corticosteroids that raise intraocular pressure; and patients who have undergone intraocular surgery.

A specific method encompassed by this embodiment is a method of treating or preventing optic neuropathy without affecting the intraocular pressure of a patient which comprises administering to a patient in need of such treatment or prevention a therapeutically effective amount of a cyclic guanosine 3',5'-monophosphate phosphodiesterase type 5 inhibitor.

Specific types of optic neuropathy that can be treated or prevented by methods encompassed by this embodiment include, but are not limited to, anterior ischemic optic neuropathy and glaucomatous optic neuropathy. Glaucomatous optic neuropathy can be caused by or associated with an acute, sub-acute, or chronic glaucoma selected from the group consisting of: chronic (idiopathic) open-angle glaucomas; pupillary block glaucomas such as acute angle-closure glaucoma, subacute angle-closure glaucoma, chronic angle-closure glaucoma, and combined-mechanism glaucoma; developmental glaucomas such as congential glaucoma, juvenile glaucoma, Axenfeld-Rieger syndrome, Peters' anomaly, and Aniridia; glaucomas associated with other ocular disorders such as disorders of the comeal endothelium, disorders of the iris and ciliary body, disorders of the lens, disorders of the retina, choroid, and vitreous, and intraocular tumors; glaucomas associated with elevated episcleral venous pressure such as systemic diseases with associated elevated intraocular pressure and glaucoma and corticosteroid-induced glaucoma; glaucomas associated with inflammation and trauma such as keratitis, episcleritis, sclentis, uveitis, ocular trauma, and hemorrhage; glaucomas following intraocular surgery such as ciliary block (malignant) glaucoma, glaucomas in aphakia and pseudoakia, epithelial, fibrous, and endothelial proliferation, glaucomas associated with comeal surgery, and glaucomas associated with vitreoretinal surgery; and low-tension glaucoma. Preferably, the acute, sub-acute, or chronic glaucoma is selected from the group consisting of: glaucomas associated with elevated episcleral venous pressure; glaucomas associated with inflammation and trauma; glaucomas following intraocular surgery; and low-tension glaucoma.

A fourth embodiment of the invention encompasses a method of treating or preventing macular (dry) degeneration which comprises administering to a patient in need of such treatment or prevention a therapeutically effective amount of a cyclic guanosine 3',5'-monophosphate phosphodiesterase type 5 inhibitor.

In each of the methods of the invention, the cyclic guanosine 3',5'-monophosphate phosphodiesterase type 5 inhibitor is preferably a compound of Formula 1:

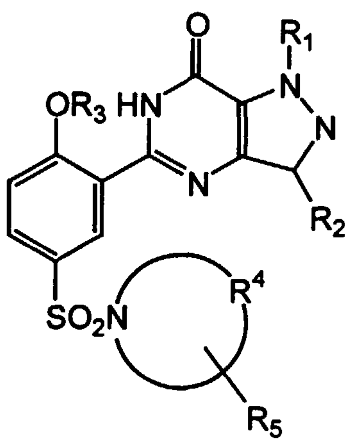

**Formula 1**

wherein:

$R^1$ is H, $C_1$-$C_3$alkyl, $C_3$-$C_5$ cycloalkyl, or perfluoroalkyl;
$R^2$ is H, $C_1$-$C_6$ alkyl optionally substituted by OH, $C_1$-$C_3$ alkoxy, or $C_3$-$C_6$ cycloalkyl, or $C_1$-$C_3$ perfluoroalkyl;
$R^3$ is $C_1$-$C_6$ alkyl, $C_3$-$C_6$ alkenyl, $C_3$-$C_6$ alkynyl, $C_3$-$C_7$ cycloalkyl, $C_1$-$C_6$ perfluoroalkyl, or ($C_3$-$C_6$ cycloalkyl)$C_1$-$C_6$ alkyl;
$R^4$ taken together with the nitrogen atom to which it is attached completes a pyrrolidinyl, piperidino, morpholino, or 4-N-($R^6$)-piperazinyl group;
$R^5$ is H, $C_1$-$C_4$ alkyl, $C_1$-$C_3$ alkoxy, $NR^7R^8$, or $CONR^7R^8$
$R^6$ is H, $C_1$-$C_6$ alkyl, ($C_1$-$C_3$ alkoxy)$C_2$-$C_6$ alkyl, hydroxy $C_2$-$C_6$ alkyl, ($R^7R^8N$)$C_2$-$C_6$ alkyl, ($R^7R^8NCO$) $C_1$-$C_6$ alkyl, $CONR^7R^8$, $CSNR^7R^8$, or $C(NH)NR^7R^8$;
$R^7$ and $R^8$ are each independently H, $C_1$-$C_4$ alkyl, ($C_1$-$C_3$ alkoxy)$C_2$-$C_4$ alkyl, or hydroxy $C_2$-$C_4$ alkyl; or a pharmaceutically acceptable salt or solvate thereof.

Preferred compounds of Formula 1 are those wherein $R^1$ is H, methyl, or ethyl; $R^2$ is $C_1$-$C_3$ alkyl optionally substituted by OH or methoxy; $R^3$ is $C_2$-$C_3$ alkyl or allyl; $R^4$ taken together with the nitrogen atom to which it is attached completes a piperidino or 4-N-($R^6$) piperazinyl group; $R^5$ is H, $NR^7R^8$, or $CONR^7R^8$; $R^6$ is H, $C_1$-$C_3$ alkyl, hydroxy $C_2$-$C_3$ alkyl, $CONR^7R^8$, $CSNR^7R^8$, or $C(NH)NR^7R^8$; and $R^7$ and $R^8$ are each independently H or methyl.

More preferred compounds of Formula 1 are those wherein $R^1$ is methyl; $R^2$ is n-propyl; $R^3$ is ethyl, n-propyl, or allyl; $R^4$ taken together with the nitrogen atom to which it is attached completes a 4-N-($R^6$) piperazinyl group; $R^5$ is H; and $R^6$ is H, $C_1$-$C_3$ alkyl, or 2-hydroxyethyl.

Even more preferred compounds of Formula 1 include:

5-[2-allyloxy-5-(4-methylpiperazinylsulphonyl)phenyl]-1-methyl-3-n-propyl-1,6-dihydro-7H-pyrazolo[4,3-d]pyrimidin-7-one;
5-[2-ethoxy-5-(piperazinylsulphonyl)phenyl]-1-methyl-3-n-propyl-1,6-dihydro-7H-pyrazolo[4,3-d]pyrimidin-7-one;
5-[2-ethoxy-5-(4-methylpiperazinylsulphonyl)pheny]-1-methyl-3-n-propyl-1,6-dihydro-7H-pyrazolo[4,3-d]pyrimidin-7-one;
5-{2-ethoxy-5-(4-(2-propyl)piperazinylsulphonyl]-phenyl}-1-methyl-3-n-propyl-1,6-dihydro-7H-pyrazolo[4,3-d]pyrimidin-7-one;
5-{2-ethoxy-5-[4-(2-hydroxyethyl)piperazinylsulphonyl]phenyl}-1 -methyl-3-n-propyl-1,6-dihydro-7H-pyrazolo[4,3-d]pyrimidin-7-one;
1-methyl-5-[5-piperazinylsulphonyl-2-n-propoxyphenyl]-3-n-propyl-1,6-dihydro-7H-pyrazolo[4,3-d]pyrimidin-7-one; and
5-{5-[4-(2-hydroxyethyl)piperazinylsulphonyl]-2-n-propoxyphenyl}-1-methyl-3-n-propyl-1,6-dihydro-7H-pyrazolo[4,3-d]pyrimidin-7-one.

A most preferred compound of Formula 1 is 5-[2-ethoxy-5-(4-methylpiperazinyl-sulphonyl)phenyl]-1-methyl-3-n-propyl-1,6-dihydro-7H-pyrazolo[4,3-d]pyrimidin-7-one. A preferred salt of this compound is the citrate salt.

In each of the methods of the invention wherein the cyclic guanosine 3',5'-monophosphate phosphodiesterase type 5 inhibitor is a compound of Formula 1 or a pharmaceutically acceptable salt or solvate thereof, the prophylactically or therapeutically effective amount of a compound of Formula 1 or a pharmaceutically acceptable salt or solvate thereof is preferably from about 5 to about 250, more preferably from about 10 to about 200, and most preferably from about 20 to about 150 mg/day.

In each of the methods of the invention, the cyclic guanosine 3',5'-monophosphate phosphodiesterase type 5 inhibitor can be administered by oral or parenteral (*e.g.*, subcutaneous, intravenous, bolus injection, intramuscular, and intraarterial) routes.

A fifth embodiment of the invention encompasses pharmaceutical dosage forms of a compound of Formula 1 or a pharmaceutically acceptable salt or solvate thereof. These dosage forms are particularly suited for use in methods of the invention. Preferred compounds of Formula 1 are provided above. A most preferred compound of Formula 1 is 5-[2-ethoxy-5-(4-methylpiperazinylsulphonyl)phenyl]-1-methyl-3-n-propyl-1,6-dihydro-7H-pyrazolo[4,3-d]pyrimidin-7-one. Dosage forms of the invention are suitable for oral and parenteral (*e.g.*, subcutaneous, intravenous, bolus injection, intramuscular, and intraarterial) administration. Preferred dosage forms of the invention comprise from about 5 to about 250, more preferably from about 10 to about 200, and most preferably from about 20 to about 150 mg of a compound of Formula 1 or a pharmaceutically acceptable salt or solvate thereof.

<u>Definitions</u>

As used herein, the term "patient" encompasses mammal, and human in particular.

As used herein, the terms "treating optic neuropathy" and "treatment of optic neuropathy" mean reversing, slowing, or preventing the advancement of optic nerve damage or disease. Symptoms of optic neuropathy usually include measurable loss of vision, which is often best noted by evaluation of the visual field.

As used herein, the term "normal intraocular pressure" means an intraocular pressure of from about 10 to about 22 mm Hg as measured with a clinically accepted tonometer or pressure taking device.

As used herein, the term "low intraocular pressure" means an intraocular pressure of less than

about 10 mm Hg as measured with a clinically accepted tonometer or pressure taking device.

As used herein, the terms "without changing intraocular pressure" and "without affecting intraocular pressure" mean that the method described does not, within error, raise or lower intraocular pressure. Error is typically about ± 2 mm Hg.

As used herein, the terms "alkyl", "alkenyl", "alkynyl", and "perfluoroalkyl" encompass straight and branched chain groups.

Detailed Description of the Invention

This invention is based on the unexpected discovery that cyclic guanosine 3',5'-monophosphate phosphodiesterase type 5 (cGMP PDE5) inhibitors such as compounds of Formula 1 may be used to prevent and/or treat diseases or disorders of the eye. The invention is thus directed to methods and compositions for the treatment or prevention of acute, sub-acute, or chronic diseases or disorders of the eye including, but are not limited to: central retinal artery occlusion; central retinal vein occlusion; optic neuropathy including, but not limited to, anterior ischemic optic neuropathy and glaucomatous optic neuropathy; and macular (dry) degeneration. The invention is further directed to methods and compositions that allow the treatment and/or prevention of diseases or conditions of the eye without significantly affecting intraocular pressure (IOP). These methods are of particular importance in the treatment or prevention of diseases or disorders of the eyes of patients with normal or low IOP.

Without being limited by theory, it is believed that the benefits provided by methods of the invention result from at least one of two different mechanisms of action. First, it is believed that the administration of a cGMP PDE5 inhibitor increases levels of cGMP which in turn increase the flow of blood to parts of the eye such as the optic nerve and retina. Advantageously, this increase in blood flow can be obtained with fewer of the adverse effects typically associated with the administration of vasodilators such as nitric oxide (NO) donors such as nitroglycerin, sodium nitrite, sodium nitroprusside, and isosorbide dinitrate. Examples of adverse effects associated with NO donors include, but are not limited to, headache, severe hypotension, and methemoglobinemia.

Second, it is believed that cGMP may have additional beneficial effects independent of its vascular effects. For example, it is possible that cGMP may have cellular effects that facilitate the treatment and/or prevention of diseases and conditions of the eye.

Inhibitors of cGMP PDE5 that can be used in the methods of the invention include, but are not limited to: a 5-substituted pyrazolo [4,3-d]pyrimidine-7-one as disclosed in European patent application 0201188; (ii) a griseolic acid derivative as disclosed in European patent applications 0214708 and 0319050; (iii) a 2-phenylpurinone derivative as disclosed in European patent application 0293063; (iv) a phenylpyridone derivative as disclosed in European patent application 0347027; (v) a fused pyrimidine derivative as disclosed in European patent application 0347146; (vi) a condensed pyrimidine derivative as disclosed in European patent application 0349239; (vii) a pyrimidopyrimidine derivative as disclosed in European patent application 0351058; (viii) a purine compound as disclosed in European patent application 0352960; (ix) a quinazolinone derivative as disclosed in European patent application 0371731; (x) a phenylpyrimidone derivative as disclosed in European patent application 0395328; (xi) an imidazoquinoxalinone derivative or an analogue thereof as disclosed in European patent application 0400583; (xii) a phenylpyrimidone derivative as disclosed in European patent application 0400799; (xiii) a phenylpyridone derivative as disclosed in European patent application 0428268; (xiv) a pyrimidopyrimidine derivative as disclosed in European patent 0442204; (xv) a 4-aminoquinazoline derivative as disclosed in European patent application 0579496; (xvi) a 4,5-dihydro4-oxo-pyrrolo[1,2-a]quinoxaline derivative or an analogue thereof as disclosed in European patent application 0584487; (xvii) a polycyclic guanine derivative as disclosed in International patent application WO 91/19717; (xviii) a nitrogenous heterocyclic compound as disclosed in International patent application WO 93/07124; (xix) a 2-benzyl-polycyclic guanine derivative as disclosed in International patent application WO 94/19351; (xx) a quinazoline derivative as disclosed in US patent 4,060,615; (xxi) a 6-heterocyclyl pyrazolo [3,4-d]pyrimidin-4-one as disclosed in US patent 5,294,612; (xxii) a benzimidazole as disclosed in Japanese patent application 5-222000; (xxiii) a cycloheptimidazole as disclosed in *European Journal of Pharmacology*, 251:1 (1994); (xxiv) a heterocycle as disclosed in International patent application WO94/22855; (xxv) a pyrazolopyrimidine derivative as disclosed in European patent application 0636626; (xxvi) a 4-aminopyrimidine derivative as disclosed in European patent application 0640599; (xxvii) a imidazoquinazoline derivative as disclosed in International patent application WO 95/06648; (xxviii) an anthranilic acid derivative as disclosed in International patent application WO 95/18097; (xxix) a 4-aminoquinazoline derivative as disclosed in US patent 5,436,233; (xxx) a tetracyclic derivative as disclosed in International patent application WO 95/19978; (xxxi) a imidazoquinazoline derivative as disclosed in European patent application 0668280; (xxxii) a quinazoline compound as disclosed in European patent application 0669324; and (xxxiii) a tetracyclic compound as disclosed in International patent application WO 95/19978 or WO 97/03675. All of the references listed above are hereby incorporated by reference.

Of particular interest for use in the present invention are compounds disclosed in EP 0579496, WO 93/07124, US 5,294,612 and WO 94/22855 (xv, xviii, xxi

and xxiv above). Additional examples of preferred compounds can be found in WO 96/16644 on pages 4-6, the contents of which are hereby incorporated by reference.

Other preferred individual compounds of the invention include 3-ethyl-5-[2-(2-methoxyethoxy)-5-(4-methylpiperazin-1-ylsulphonyl)pyridin-3-yl]-2-(pyridin-2-yl)methyl-2,6-dihydro-7H-pyrazolo[4,3-d]pyrimidin-7-one; 3-ethyl-5-[5-(4-ethylpiperazin-1-ylsulphonyl)-2)2-methoxyethoxy)pyridin-3-yl]-2-(pyridin-2-yl)methyl-2,6-dihydro-7H-pyrazolo[4,3-d]pyrimidin-7-one; 3-ethyl-5-[5-(4-ethyl-4-oxidopiperazin-1-ylsulphonyl)-2-(2-methoxyethoxy)pyridin-3-yl)-2-(pyridin-2-yl)methyl-2,6-dihydro-7H-pyrazolo[4,3-d]pyrimidin-7-one; 5-[2-(2-methoxyethyoxy)-5-(4-methylpiperazin-1-ylsulphonyl)pyridin-3-yl]-3-n-propyl-2-(pyridin-2-yl)methyl-2,6-dihydro-7H-pyrazolo[4,3-d]pyrimidin-7-one; 5-[5-(4-ethylpiperazin-1-ylsulphonyl)-2-(2-methoxyethoxy)pyridin-3-yl]-3-n-propyl-2-(pyridin-2-yl)methyl-2,6-dihydro-7H-pyrazolo[4,3-d]pyrimidin-7-one; (+)-3-ethyl-5-[5-(4-ethylpiperazin-1-ylsulphonyl)-2-(2-methoxy-1(R)-methylethoxy)pyridin-3-yl]-2-methyl-2,6-dihydro-7-Hpyrazolo[4,3-d]pyrimidin-7-one; 3-ethyl-5-[5-(4-ethylpiperazin-1-ylsulphonyl)-2-(2-methoxy-1(R)-methylethoxy)pyridin-3-yl]-2-(6-methylpyridin-2-yl)methyl-2,6-dihydro-7H-pyrazolo[4,3-d]pyrimidin-7-one; 5-[2-ethoxy-5-(4-ethylpiperazin-1-ylsulphonyl)pyridin-3-yl]-3-ethyl-2-(6-methoxypyridin-2-yl)methyl-2,6-dihydro-7H-pyrazolo[4,3-d]pyrimidin-7-one; 5-[2-i-butoxy-5-(4-ethylpiperazin-1-ylsulphonyl)pyridin-3-yl]-2,3-diethyl-2,6-dihydro-7H-pyrazolo[[4,3-d]pyrimidin-7-one; and 5-[2-ethoxy-5-(4-ethylpiperazin-1-ylsulphonyl)pyridin-3-yl]-3-ethyl-2-[1-pyridin-2-yl)ethyl]2,6-dihydro-7H-pyrazolo[4,3-d]pyrimidin-7-one.

Most preferred cGMP PDE5 inhibitors are of Formula 1. These compounds can be prepared according to methods disclosed by U.S. Patent No. 5,250,534, which is incorporated herein by reference. Compounds of Formula 1 can contain one or more asymmetric centers, and can thus exist as enantiomers or diastereoisomers: these isomers, and mixtures of these isomers, can be used in the methods and compositions of the invention. Compounds of Formula 1 can also exist in tautomeric forms which can be used in the methods and compositions of the invention. Radiolabeled derivatives of compounds of Formula 1 can be used in the methods and compositions of the invention if so desired.

Pharmaceutically acceptable salts and solvates of compounds of Formula 1 can be used in the methods and can be incorporated into the dosage forms of the invention. Pharmaceutically acceptable salts of compounds of Formula 1 which contain a basic center are acid addition salts formed with pharmaceutically acceptable acids. Examples include, but are not limited to, hydrochloride, hydrobromide, sulphate or bisulphate, phosphate or hydrogen phosphate, acetate, citrate, fumarate, gluconate, lactate, maleate, succinate, and tartrate salts. Compounds of the Formula 1 can also provide pharmaceutically acceptable metal salts, particularly alkali metal salts, with bases. Examples include, but are not limited to, sodium and potassium salts. A preferred salt of compounds of Formula 1 is the citrate salt.

## Pharmaceutical Formulations and Methods of Treatment

Compounds of Formula 1 and their pharmaceutically acceptable salts and solvates (hereinafter referred to as the "compounds of the invention") can be administered to a patient by various routes. These include, but are not limited to, oral and parenteral (*e.g.*, subcutaneous, intravenous, bolus injection, intramuscular, and intraarterial) routes. Capsules or tablets can be used for oral administration, and sterile solutions or suspensions can be used for parenteral administration.

A preferred daily dose of a compound of the invention depends on a variety of factors well known to the skilled clinician, including the specific compound administered, the route of administration, and the individual response of the patient. The size of a dose can also depend upon whether the condition being treated is chronic, sub-acute, or acute. Generally, however, a preferred daily dose for the treatment or prevention of a disease or condition of the eye ranges from about 5 to about 250 mg/day, more preferably from about 10 to about 200 mg/day, and most preferably from about 20 to about 150 mg/day for the average adult, and may be administered in a single or divided doses. These dosages and dose frequencies are encompassed by the phrases "prophylactically effective" and "therapeutically effective" as used herein.

The compounds of the invention can be administered orally with an inert diluent or an edible carrier, or can be enclosed in gelatine capsules or compressed into tablets. Such preparations typically contain at least 0.1% of a compound of the invention. A typical oral dosage unit contains from about 5 mg to about 250 mg of a compound of the invention.

Compounds of the invention can be administered alone or in combination with pharmaceutically acceptable carriers or diluents by the routes previously indicated. Such administration can be carried out in single or multiple doses. The compounds can be administered in a wide variety of different dosage forms, *i.e.*, they can be combined with various pharmaceutically acceptable inert carriers in the form of tablets, capsules, lozenges, aqueous suspensions, injectable solutions, elixirs, syrups, and the like. Such carriers include solid diluents or fillers, sterile aqueous media and various non-toxic organic solvents. Moreover, oral pharmaceutical compositions can be suitably sweetened and/or flavored. In general, the compounds are present in such dosage forms at concentration levels ranging from about 5.0% to about 70% by weight.

For oral administration, tablets containing various excipients such as microcrystalline cellulose,

sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Lubricating agents, surfactants, and glidants such as magnesium stearate, sodium lauryl sulfate, and talc are also useful for tableting purposes. Solid compositions of a similar type may also be employed as fillers in gelatin capsules. Preferred fillers include lactose or milk sugar as well as high molecular weight polyethylene glycols. When aqueous suspensions and/or elixirs are desired for oral administration, the compound may be combined with various sweetening or flavoring agents, coloring matter or dyes, and, if so desired, emulsifying and/or suspending agents as well, together with diluents such as water, ethanol, propylene glycol, glycerin and various like combinations thereof.

In addition to the common dosage forms set out above, the compounds of the invention can be administered by controlled release means and/or delivery devices capable of releasing the compound at the required rate to maintain constant pharmacological activity for a desirable period of time. Such dosage forms provide a supply of a drug to the body during a predetermined period of time, and thus maintain drug levels in the therapeutic range for longer periods of time than conventional non-controlled formulations. Suitable controlled release pharmaceutical compositions and delivery devices that may be adapted for the administration of the compounds of the invention are described by U.S. Patent Nos.: 3,847,770; 3,916,899; 3,536,809; 3,598,123; 3,630,200; 4,008,719; 4,687,610; 4,769,027; 5,674,533; 5,059,595; 5,591,767; 5,120,548 ; 5,073,543; 5,639,476; 5,354,566; and 5,733,566, the disclosures of which are hereby incorporated by reference. For example, the compounds can be coupled to a class of biodegradable polymers useful in achieving controlled release of a drug, for example, polylactic acid, polyglycolic acid, copolymers of polylactic and polyglycolic acid, polyepsion caprolatone, polyhydroxy butyric acid, copolymers of polylactic and polyglycolic acid, polyepsilon caprolactone, polyhydroxy butyric acid, polyorthoesters, polyacetals, polydihydropyrans, polycyanoacrylates and cross-linked or amphipathic block copolymers of hydrogels.

Aqueous and non-aqueous solutions, and emulsions and mixtures thereof can be used for parenteral administration of the compounds of the invention. For example, a compound of the invention can be dissolved in an oil, such as sesame or peanut oil, in water, or in aqueous propylene glycol. Although not always necessary, aqueous solutions can be suitably buffered as is known in the art. Liquid diluents are preferably rendered isotonic prior to use. These aqueous solutions are suitable for intravenous injection purposes. The oily solutions are suitable, for example, for intraarticular, intramuscular, and subcutaneous injection. The preparation of all these solutions under sterile conditions is readily accomplished by standard pharmaceutical techniques well known to those skilled in the art.

The compounds can also be coupled with soluble polymers as targetable drug carriers. Such polymers can include polyvinlpyrrolidone, pyran copolymer, polyhydroxypropylmethacrylamide phenyl, polyhydroxyethylaspartamide-phenol, or polyethyleneoxide-polylysine substituted with palmitoyl residues.

Further novel aspects of the invention are described in the example which follows.

<u>Example 1: Determining the Effect on Blood Flow to the Optic Nerve</u>

Effects of the compounds of the invention on blood flow to the optic nerve are preferably determined using laser doppler flowmetry (LDF), which can measure blood flow in the optic nerve head (ONFlow) and choroid (ChFlow). A study of the effects of orally administered sildenafil citrate on ONFlow is as follows.

Twelve healthy adults without histories of systemic or intraocular disease and who are not taking systemic or topical medications are used. Corrected visual acuities are preferably 20/20 and IOPs are preferably less than about 20 mm Hg. Slit lamp and dilated fundascopic examinations should be normal in all patients. On the days when the experiments are carried out, participants are asked to abstain from smoking and drinking caffeine-containing drinks until the end of the measurements.

Pupils are dilated with tropicamide 1% (Alcon, Huxnacao, Puerto Rico), and Polaroid (Cambridge, MA) color fundus photographs of the discs are taken for accurate documentation of the location of the blood flow measurements. Laser doppler flowmetry is used to assess relative blood velocity, volume and flow in the optic nerve head and choroid. All measurements are performed in one eye (the study eye), which is determined at random.

Laser doppler flowmetry measurements are obtained with the Oculix LDF instrument (Philadelphia, PA) based on a modified Topcon TRCJE fundus camera (Tokyo, Japan) which allows the delivery of a laser beam to any point of the posterior pole. A diode laser (670 nm) is used.

Analysis of the data is performed using software specifically developed for the analysis of doppler signals from ocular tissues. Detailed descriptions of the technique have been previously published. See, e.g., Riva, C.E., *et al*., *Invest*. *Ophthalmol*. *Vis*. *Sci*. 35:4273-4281 (1994); Riva, C.E., *et al*., *Exp. Eye Res*. 55:499-506 (1992); and Petrig, B.L. and Riva, C.E., *Ocular Blood Flow*, Kaiser, H.J. *et al*. eds., pp. 120-127 (1996). In brief, relative choroidal blood velocity (ChVel), volume (ChVol), and flow (ChFlow) are assessed by aiming the

probing laser beam at the fovea. Subsequently, LDF measurements of optic nerve head relative blood velocity, volume, and flow are obtained from the superior temporal and the inferior temporal neuroretinal rim in areas devoid of large vessels. Determinations at these two locations are averaged to provide a mean optic nerve head velocity (ONVel), volume (ONVol), and flow (ONFlow). Measurements obtained with this technique are estimated to reflect contributions not only from the superficial and prelaminar regions of the optic nerve head, but also from part of the laminar vessels.

Immediately after LDF determinations, the systolic and diastolic blood pressures are measured by sphygmomanometry and the heart rate is determined. After instillation of two drops of topical proparacaine HCl 0.5% (Allergan Pharmaceuticals, Hormigueros, Puerto Rico), IOP is measured in both eyes by Goldman applanation tonometry. Mean brachial artery blood pressure (BPm) is calculated according to the formula $Bpm = 2/3BPd + 1/3BPs$ , where BPd and BPs are the brachial artery diastolic and systolic pressures, respectively. Perfusion pressure (PP) is estimated as $PP = 2/3BPm - IOP$ .

In a double-masked, randomized, cross-over design, each subject receives orally either 50 mg of sildenafil citrate or placebo. ChFlow and ONFlow are determined monocularly at baseline and one hour after dosing. Additional measurements at two, three, and four hours can also be obtained. Mean arterial blood pressure, heart rate and intraocular pressure are monitored, and ocular perfusion pressure is estimated using known techniques. Determination of ONVel, ONVol, ONFlow, ChVel, ChVol, and ChFlow are performed by one trained examiner, masked with regard to treatment regimen. Results are expressed as mean percentage variations from baseline (± SEM). Normal distribution of the data is assessed with the Wilk-Shapiro test. Statistical evaluation of the results is performed using two-tailed, paired Student*s t-test, linear regression, and correlation analysis. Probability values <0.05 are considered statistically significant.

The present invention is not to be limited by the disclosure provided above, and its scope is further defined by the claims appended hereto.

## Claims

1. The use of a cyclic guanosine 3',5'-monophosphate phosphodiesterase type 5 inhibitor for the manufacture of a medicament for treating or preventing central retinal or posterior ciliary artery occlusion; central retinal vein occlusion; optic neuropathy; or macular (dry) degeneration.

2. The use according to claim 1 wherein the cyclic guanosine 3',5'-monophosphate phosphodiesterase type 5 inhibitor is a compound of Formula 1:

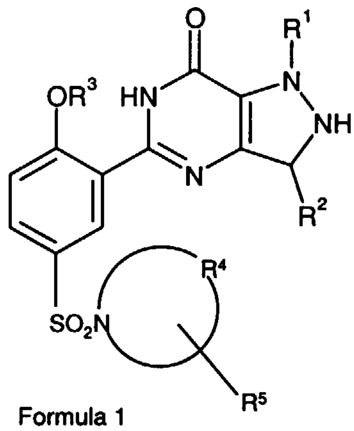

Formula 1

wherein:

$R^1$ is H, $C_1$-$C_3$ alkyl, $C_3$-$C_5$ cycloalkyl, or perfluoroalkyl;
$R^2$ is H, $C_1$-$C_6$ alkyl optionally substituted by OH, $C_1$-$C_3$ alkoxy, or $C_3$-$C_6$ cycloalkyl, or $C_1$-$C_3$ perfluoroalkyl;
$R^3$ is $C_1$-$C_6$ alkyl, $C_3$-$C_6$ alkenyl, $C_3$-$C_6$ alkynyl, $C_3$-$C_7$ cycloalkyl, $C_1$-$C_6$ perfluoroalkyl, or ($C_3$-$C_6$ cycloalkyl)$C_1$-$C_6$ alkyl;
$R^4$ taken together with the nitrogen atom to which ills attached completes a pyrrolidinyl, piperidino, morpholino, or 4-N-($R^6$)-piperazinyl group;
$R^5$ is H, $C_1$-$C_4$ alkyl, $C_1$-$C_3$ alkoxy, $NR^7R^8$, or $CONR^7R^8$;
$R^6$ is H, $C_1$-$C_6$ alkyl, ($C_1$-$C_3$ alkoxy)$C_2$-$C_6$ alkyl, hydroxy $C_2$-$C_6$ alkyl, ($R^7R^8N$)$C_2$-$C_6$alkyl, ($R^7R^8NCO$) $C_1$-$C_6$ alkyl, $CONR^7R^8$, $CSNR^7R^8$, or $C(NH)NR^7R^8$ and
$R^7$ and $R^8$ are each independently H, $C_1$-$C_4$ alkyl, ($C_1$-$C_3$ alkoxy)$C_2$-$C_4$ alkyl, or hydroxy $C_2$-$C_4$ alkyl; or a pharmaceutically acceptable salt or solvate thereof.

3. The use according to claim 2 wherein $R^1$ is H, methyl, or ethyl; $R^2$ is $C_1$-$C_3$ alkyl optionally substituted by OH or methoxy; $R^3$ is $C_2$-$C_3$ alkyl or allyl; $R^4$ taken together with the nitrogen atom to which it is attached completes a piperadino or 4-N-($R^6$)-piperazinyl group; $R^5$ is H, $NR^7R^8$, or $CONR^7R^8$;$R^6$ is H, $C_1$-$C_3$ alkyl, hydroxy $C_2$-$C_3$ alkyl, $CONR^7R^8$, $CSNR^7R^8$, or $C(NH)NR^7R^8$;and $R^7$ and $R^8$ are each independently H or methyl.

4. The use according to claim 3 wherein $R^1$ is methyl; $R^2$ is n-propyl; $R^3$ is ethyl, n-propyl, or allyl; $R^4$ taken together with the nitrogen atom to which it is attached completes a 4-N-($R^6$)piperazinyl group: $R^5$ is H; and $R^6$ is H, $C_1$-$C_3$ alkyl, or 2-hydroxyethyl.

**5.** The use according to claim 4 wherein the compound of Formula 1 is selected from the group consisting of:

5-[2-allyloxy-5-(4-methylpiperazinylsulphonyl)phenyl]-1-methyl-3-n-propyl-1,6-dihydro-7H-pyrazolo[4,3-d]pyrimidin-7-one;
5-[2-ethoxy-5-(piperazinylsulphonyl)phenyl]-1-methyl-3-n-propyl-1,6-dihydro-7H-pyrazolo[4,3,-d]pyrimidin-7-one;
5-[2-ethoxy-5-(4-methylpiperazinylsulphonyl)phenyl]-1-methyl-3-n-propyl-1,6-dihydro-7H-pyrazolo[4,3-d]pyrimidin-7-one;
5-{2-ethoxy-5-(4-(2-propyl)piperazinylsulphonyl]-phenyl}-1-methyl-3-n-propyl-1,6-dihydro-7H-pyrazolo[4,3-d]pyrimidin-7-one;
5-{2-ethoxy-5-[4-(2-hydroxyethyl)piperazinylsulphonyl]phenyl}-1-methyl-3-n-propyl-1,6-dihydro-7H-pyrazolo[4,3-d]pyrimidin-7-one;
1-methyl-5-[5-piperazinylsulphonyl-2-n-propoxy-phenyl]-3-n-propyl-1,6-dihydro-7H-pyrazolo[4,3-d]pyrimidin-7-one; and
5-{5-[4-(2-hydroxyethyl)piperazinylsulphonyl]-2-n-propoxyphenyl}-1-methyl-3-n-propyl-1,6-dihydro-7H-pyrazolo[4,3,-d]pyrimidin-7-one.

**6.** The use according to claim 5 wherein the compound of Formula 1 is 5-[2-ethoxy-5-(4-methylpiperazinyl-sulphonyl)phenyl]-1-methyl-3-n-propyl-1,6-dihydro-7H-pyrazolo[4,3,d]pyrimidin-7-one.

**7.** The use according to claim 1 wherein the cyclic guanosine 3',5'-monophosphate phosphodiesterase type 5 inhibitor is 5-[2-ethoxy-5-(4-methylpiperazinyl-sulphonyl)phenyl]-1-methyl-3-n-propyl-1,6-dihydro-7H-pyrazolo[4,3-d]pyrimidin-7-one citrate.

**8.** The use according to claim 7 wherein the medicament comprises 5 to 250 mg/day.

**9.** The use according to claim 7 wherein the medicament comprises 10 to 200 mg/day.

**10.** The use according to claim 7 wherein the medicament comprises 20 to 150 mg/day.

**11.** The use according to claim 1 wherein when treating or preventing optic neuropathy the patient group is selected from the group consisting of: patients with elevated intraocular pressure; patients greater than about 50 years of age; patients with family histories of optic neuropathy; patients with hypertension; patients with diabetes; patients with family histories of diabetes or heart disease; patients who have used, or are currently using, corticosteroids that raise intraocular pressure; and patients who have undergone intraocular surgery.

**12.** The use according to claim 1 wherein the optic neuropathy is glaucomatous optic neuropathy.

**13.** The use according to claim 12 wherein the glaucomatous optic neuropathy is caused or associated with an acute, sub-acute, or chronic glaucoma selected from the group consisting of: chronic (idiopathic) open-angle glaucomas; pupillary block glaucomas; developmental glaucomas; glaucomas associated with other ocular disorders; glaucomas associated with elevated episcleral venous pressure; glaucomas associated with inflammation and; glaucomas following intraocular surgery; and low-tension glaucoma.

**14.** The use according to claim 13 wherein the acute, sub-acute, or chronic glaucoma is selected from the group consisting of: glaucomas associated with elevated episcleral venous pressure; glaucomas associated with inflammation and trauma; glaucomas following intraocular surgery; and low-tension glaucoma.

**15.** The use according to claim 1 wherein the optic neuropathy is anterior ischemic optic neuropathy.